# EUROPEAN PATENT APPLICATION

(11) **EP 4 714 401 A1**
(43) Date of publication of application: **25.03.2026**
(21) Application number: 24806462.8
(22) Date of filing: 10.05.2024
(51) Int. Cl.: A61F 2/82, A61F 2/07, A61F 2/95

(54) **STENT, INNER CATHETER, CONVEYING ASSEMBLY, AND CONVEYING DEVICE**

(30) Priority: 18.05.2023 CN 202310565736
(71) Applicant: Shanghai MicroPort Endovascular MedTech (Group) Co., Ltd., Shanghai 201318 (CN)
(72) Inventor: WU, Weiyi, Shanghai 201318 (CN); ZHU, Yongfeng, Shanghai 201318 (CN); GAO, Yang, Shanghai 201318 (CN); YAO, Yu, Shanghai 201318 (CN); SHAN, Wenwen, Shanghai 201318 (CN)
(74) Representative: Murgitroyd & Company
(86) International application number: PCT/CN2024/092230
(87) International publication number: WO 2024/235110

(57) **Abstract**

The present application relates to a stent, an inner catheter, a delivery assembly, and a delivery device. The stent includes a stent body (1000) and a first constraining member (2000). The stent body (1000) has a stent lumen extending therethrough in an axial direction, and a stent window (1100) communicating with the stent lumen is defined on a surface of the stent body (1000). The tent body 1000) includes a constrained section (1000a) and a non-constrained section (1000b) distributed along the axial direction, and the stent window (1100) is located between the constrained section (1000a) and the non-constrained section (1000b). The first constraining member (2000) is disposed on the constrained section (1000a) of the stent body (1000).

## Description

### CROSS-REFERENCE TO RELATED APPLICATIONS

The present application claims priority to Chinese patent application No. 202310565736.2, filed on May 18, 2023, and entitled "STENT, INNER CATHETER, DELIVERY ASSEMBLY AND DELIVERY DEVICE", the entire content of which is incorporated herein by reference.

### TECHNICAL FIELD

The present disclosure relates to the field of medical instruments technologies, and in particular, to a stent, an inner catheter, a delivery assembly, and a delivery device.

### BACKGROUND

With the continuous advancement of endovascular treatment techniques and interventional devices, endovascular aortic repair (EVAR) has gained widespread acceptance due to its advantages of being minimally invasive, reducing mortality, decreasing perioperative morbidity, and shortening hospital stays. It has gradually replaced conventional open surgery and become the preferred treatment for Stanford Type B aortic dissection. As surgeons deepen their understanding of EVAR, many are now employing in-situ fenestration techniques or pre-fenestration techniques on straight tubular stents, or using integrated branched stent graft systems to reconstruct important branch vessels, thereby expanding the applicability of EVAR. However, regardless of the stent implantation method used, establishing access to branch vessels remains crucial.

For in-situ fenestration techniques, most utilize puncture needles or endoluminal laser perforation technology to create holes in the straight tubular stents, followed by balloon dilation to complete the fenestration. Branch vessels are then super-selected through this fenestration, and then small branch stents are implanted to ensure blood supply to the branch vessels. For pre-fenestration techniques, manufacturers often pre-incorporate fenestrations during the stent manufacturing process. Alternatively, some surgeons perform on-site fenestration on the stent during the procedure, then reinstall the stent and implant it into the patient's body. After stent implantation, a guide catheter and guidewire are used to thread through the pre-fenestrated site of the main stent, select into the branch vessel, thus establishing a pathway, and then guide the placement of the small branch stent into the target vessel. Furthermore, for integrated branched stent systems, the entire stent is positioned near the branch vessel. After pulling the integrated branch stent into the branch vessel, the entire stent system is fully deployed.

However, in-situ fenestration techniques have certain uncertainties during the procedure. Due to the unpredictable morphology of the stent during deployment, the branch vessel orifice is often obstructed by metal stent segments. This obstruction may not be clearly identifiable under angiography, potentially leading to direct damage to the metal stent segments during perforation, or significant deviation of the perforation site due to stent interference, even causing vascular injury. In pre-fenestration techniques, a fenestrated main stent is typically implanted first, followed by super-selective establishment of guiding pathways for branch vessels. This process can be compromised by inaccurate pre-operative angiographic assessment or intra-operative positioning issues. After the main stent is deployed, the stent segment springs open, making it difficult to accurately align the fenestration region with the branch vessel orifice. Some branch vessels may be covered by the graft membrane, significantly increasing the difficulty of super-selection using guidewires and catheters. Integrated branched stent systems often require pre-guidance for the branch stent and are generally limited to specific vascular regions. For branch vessels in organ regions, they cannot pull the branch stents into position. Therefore, there is an urgent need in this field to provide a simple and reliable method for establishing access to branch vessels.

### SUMMARY

Therefore, it is necessary to provide a stent, an inner catheter, a delivery assembly, and a delivery device to address at least one of the aforementioned technical problems.

In a first aspect, a stent is provided in the present disclosure. The stent includes:
a stent body having a stent lumen extending therethrough in an axial direction, a stent window communicating with the stent lumen being defined on a surface of the stent body, the stent body having an expanded state and a constrained state, the stent body in the expanded state including a constrained section and a non-constrained section distributed along the axial direction, and the stent window being located at a junction between the constrained section and the non-constrained section when the stent body is in the constrained state; and
a first constraining member disposed on the constrained section of the stent body and configured to assist in constraining the constrained section of the stent body, such that the constrained section of the stent body is partially expanded.

In an embodiment, the stent body includes two non-constrained sections and one constrained section distributed along the axial direction, the constrained section is located between the two non-constrained sections, the stent window includes two stent windows, and the two stent windows are located at two junctions between the two non-constrained sections and the constrained section, respectively.

In an embodiment, the first constraining member includes two first constraining members, and the two first constraining members are spaced apart along the axial direction on the constrained section.

In an embodiment, the first constraining member is a first constraining coil connected to the constrained section of the stent body.

In an embodiment, the first constraining coil includes two unit coils, both of which are connected to the constrained section of the stent body. One of the unit coils in the first constraining coil is configured to thread through the other unit coil, forming a constraining semi-annular channel in a portion of the unit coil threaded through the other unit coil.

In a second aspect, an inner catheter is provided in the present disclosure. The inner catheter includes:
an inner tube body having an inner tube lumen extending therethrough in an axial direction, and having a stent mounting section configured to mount the stent of any one of the above embodiments; and
a second constraining member disposed on the inner tube body and configured to assist in constraining the stent.

In an embodiment, the second constraining member is provided with a constraining threading hole configured for a constraining wire to pass through.

In an embodiment, the second constraining member includes two second constraining members, and the two constraining members are spaced apart along the axial direction on the stent mounting section.

In an embodiment, the second constraining member is a second constraining coil connected to the stent mounting section of the inner tube body.

In a third aspect, a delivery assembly is provided in the present disclosure. The delivery assembly includes:
an outer catheter having an outer tube lumen extending therethrough in an axial direction;
the inner catheter of any one of the above embodiments, the inner catheter being movably assembled within the outer tube lumen of the outer catheter;
the stent of any one of the above embodiments, the stent being assembled between the outer catheter and the inner catheter, and the stent being located at the stent mounting section of the inner catheter; and
a constraining assembly configured to connect to the first constraining member, thus constraining the constrained section of the stent body onto the inner catheter, such that the constrained section of the stent body is partially expanded.

In an embodiment, the constraining assembly includes the second constraining member on the inner catheter and a third constraining member.

In an embodiment, the third constraining member is a constraining wire. The constraining wire is threaded through the stent window of the stent and connected to the first constraining member and the second constraining member, thus constraining the constrained section of the stent body onto the inner catheter, such that the constrained section of the stent body is partially expanded.

In a fourth aspect, a delivery device is provided in the present disclosure. The delivery device includes the delivery assembly described in any one of the above embodiments.

In the aforementioned stent, inner catheter, delivery assembly, and delivery device, the stent can implement a semi-constrained technique after being implanted in the body, where the non-constrained section of the stent freely expands while the constrained section is controllably partially expanded. This semi-constraining technique mentioned above can simplify the operation for stent fenestration, which can both ensure blood supply in the main lumen and facilitate the super-selection of the stent window and branch vessels. Moreover, the non-constrained section of the stent has already conformed to the blood vessel, so the stent will not easily shift during subsequent super-selection. The clamping technique of the stent also provides more operability for super-selection.

### BRIEF DESCRIPTION OF THE DRAWINGS

To more clearly illustrate the technical solutions in the embodiments or related technologies of the present disclosure, the accompanying drawings used in the description of the embodiments or related technologies will be briefly introduced below. Obviously, the accompanying drawings described below are only some embodiments of the present disclosure. For those skilled in the art, other drawings can be obtained according to these drawings without making any creative efforts.
FIG. 1 is a schematic diagram of a stent in a contracted state according to an embodiment of the present disclosure.
FIG. 2 is a schematic diagram of a stent in a semi-constrained released state according to an embodiment of the present disclosure.
FIG. 3 is a schematic diagram of a connection structure of a first constraining coil including two unit coils according to an embodiment of the present disclosure.
FIG. 4 is a schematic diagram of a structure of a delivery assembly according to an embodiment of the present disclosure.
FIG. 5 is a schematic diagram of a structure of a second constraining member implemented as a second constraining coil according to an embodiment of the present disclosure.
FIG. 6 is a schematic diagram illustrating a usage state of a delivery device at a first stage according to an embodiment of the present disclosure.
FIG. 7 is a schematic diagram illustrating a usage state of the delivery device at a second stage according to an embodiment of the present disclosure.

Reference signs:
1000, Stent body; 2000, First constraining member; 3000, Inner tube body; 4000, Second constraining member; 5000, Outer catheter; 6000, Third constraining member;
1100, Stent window; 1000a, Constrained section; 1000b, Non-constrained section;
2000a, Unit coil.

### DETAILED DESCRIPTION OF THE EMBODIMENTS

In order to make the above objectives, features and advantages of the present disclosure more obvious and understandable, specific implementations of the present disclosure are described in detail below with reference to the accompanying drawings. In the following description, many specific details are set forth in order to fully understand the present disclosure. However, the present disclosure can be implemented in many other ways different from those described herein, and those skilled in the art can make similar improvements without departing from the connotation of the present disclosure. Therefore, the present disclosure is not limited by the specific embodiments disclosed below.

In the description of the present disclosure, it should be understood that if the terms "center", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", etc. are used, these terms indicate orientations or position relationships as shown in the accompanying drawings and are merely intended to facilitate the description of the present disclosure and simplify the description, rather than indicating or implying that the indicated device or element must have a particular orientation or be constructed and operated in a particular orientation, and therefore these terms should not to be understood as a limitation on the present disclosure.

Furthermore, if the terms such as "first" and "second" are used, they are used for descriptive purposes only, and should not be understood as indicating or implying relative importance or implicitly indicating the quantity of the technical features indicated. Thus, the features described with "first" and "second", etc., may explicitly or implicitly include at least one of these features. In the description of the present disclosure, if the term "plurality" is used, it means at least two, such as two, three, etc., unless otherwise clearly and specifically defined.

In the present disclosure, unless otherwise clearly specified and limited, if the terms "mount", "couple", "connect", "fix", etc., are used, these terms should be understood in a broad sense, for example, it may be a fixed connection, a detachable connection, or integration. It may be a mechanical connection or an electrical connection. It may be a direct connection or an indirect connection through an intermediate medium. It may be an internal connection between two elements or an interaction relationship between the two elements, unless otherwise clearly defined. For a person of ordinary skill in the art, the specific meanings of the above terms in the present disclosure can be understood according to specific circumstances.

In the present disclosure, unless otherwise clearly specified and limited, if there is a description that a first feature is "on" or "under" a second feature, etc., it may mean that the first and second features are in direct contact, or the first and second features are in indirect contact through an intermediate medium. Moreover, the first feature being "on", "above", or "over" the second feature may mean that the first feature is directly above or obliquely above the second feature, or simply means that the horizontal height of the first feature is greater than that of the second feature. The first feature being "under", "beneath" and "below" the second feature may mean that the first feature is directly below or obliquely below the second feature, or simply means that the horizontal height of the first feature is less than the that of second feature.

It should be noted that if an element is referred to as being "fixed to" or "disposed on" another element, it can be directly on the other element or there may be an intervening element. If an element is considered to be "connected to" another element, it can be directly connected to the other element or there may be an intervening element. If so, the terms "vertical", "horizontal", "upper", "lower", "left", "right" and similar expressions used in the present disclosure are for the purpose of illustration only and do not represent the only possible implementation.

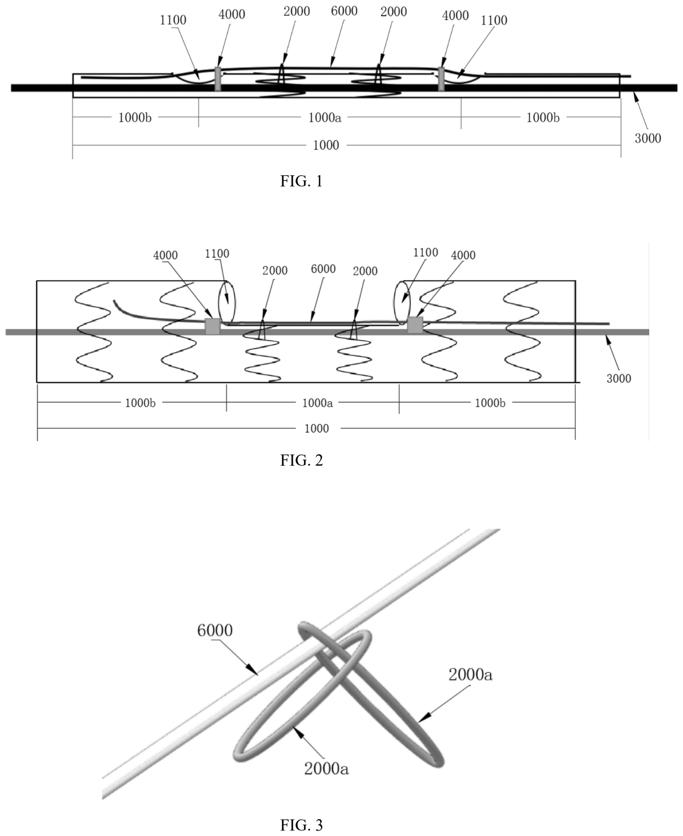

Referring to FIG. 1, an embodiment of the present disclosure provides a stent, which includes a stent body 1000 and a first constraining member 2000. The stent body 1000 has a stent lumen extending therethrough in an axial direction. A stent window 1100 communicating with the stent lumen is provided on a surface of the stent body 1000. The stent body 1000 includes a constrained section 1000a and a non-constrained section 1000b distributed along the axial direction. The stent window 1100 is located between the constrained section 1000a and the non-constrained section 1000b. In some embodiments, the stent body 1000 has an expanded state and a constrained state. The stent body 1000 in the expanded state includes the constrained section 1000a and the non-constrained section 1000b distributed along the axial direction. The stent window 1100 is located at a junction between the constrained section 1000a and the non-constrained section 1000b when the stent body 1000 is in the constrained state. The first constraining member 2000 is disposed on the constrained section 1000a of the stent body 1000 and is configured to assist in constraining the constrained section 1000a of the stent body 1000, such that the constrained section 1000a of the stent body 1000 is partially expanded. This allows for adjustment of a position of the stent window 1100 to align it with a position of a branch vessel. After the adjustment is completed, the stent body 1000 is allowed to be fully released.

The stent body 1000 can be a tubular structure or similar structure, such as having a stent lumen extending therethrough in an axial direction, and a distal stent port and a proximal stent port at both ends of the stent communicating with the stent lumen. The stent window 1100 provided on the surface of the stent body 1000 can communicate with its stent lumen, enabling the stent window 1100 not only to meet the super-selection requirement of branch vessels but also to allow other cooperating constraining structures of the first constraining member 2000 to smoothly thread through the stent lumen and the stent window 1100 of the stent body 1000, thus cooperating with the first constraining member 2000 to constrain the constrained section 1000a of the stent body 1000.

The numbers of the constrained section(s) 1000a, the stent window(s) 1100, and the first constraining member(s) 2000 on the stent body 1000 can be determined according to actual conditions, and are not limited in this embodiment. Since the constrained section 1000a occupies a certain region in the stent body 1000, it typically contains multiple stent segments of the stent body 1000. The number of the first constraining member(s) 2000 can correspond to a number of stent segments contained within the constrained section 1000a. For example, as shown in FIGS. 1 and 2, the number of first constraining member 2000 corresponds one-to-one with the number of stent segments for constrained matching, utilizing one first constraining member 2000 to ligate and constrain one stent segment, effectively pressing down the stent segment. Therefore, the number of the constrained section(s) 1000a can be one, two, three or more. The number of the stent segments contained within a constrained section 1000a can be one, two, three or more. The number of the stent window(s) 1100 can be one, two, three or more. Correspondingly, the number of the first constraining member(s) 2000 can also be one, two, three or more. Those skilled in the art can adjust the matching numbers according to requirements, which are not limited here.

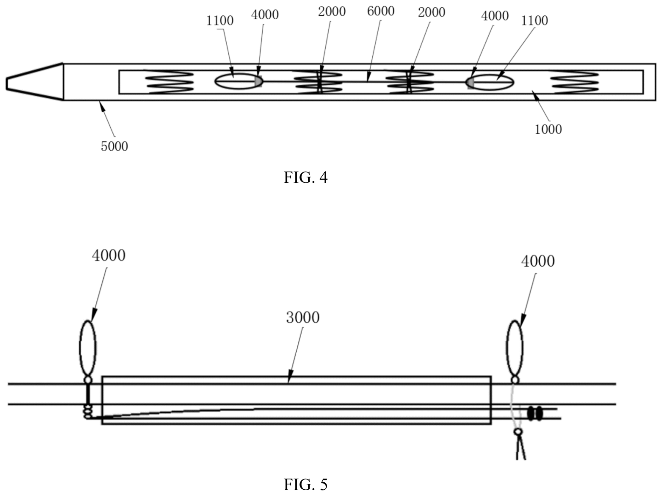

The stent provided in the present disclosure also belongs to the type that can be delivered into the human body using a delivery device for surgical treatment. However, the stent provided in the present disclosure, based on improvements in structure and usage, allows for flexible control of the intended contracted state and released state. The delivery device typically includes an outer catheter 5000 and an inner catheter (as shown in FIG. 4). The stent can be sleeved over the exterior of the inner catheter and housed inside the outer catheter 5000. A radial inward force applied by the outer catheter 5000 on the stent body keeps the stent body in a contracted state, stably assembled between the inner catheter and the outer catheter 5000 in the contracted state. Therefore, when the outer catheter 5000 is removed, the stent body, having lost the radial inward force applied by the outer catheter 5000, can expand and transition from the contracted state to a released state.

The contracted state of the stent body refers to a state where the stent body retracts into a smaller form due to external forces, such as the radial inward force applied by the outer catheter 5000. The released state of the stent body refers to a state where the stent body expands into a larger form when not subjected to external forces. However, the stent provided in the present disclosure can be maintained in a semi-constrained state between the contracted state and the released state. The semi-constrained released state means that when the stent expands naturally upon removal of the radial inward force applied by the outer catheter 5000, the non-constrained section 1000b of the stent can expand normally, while the constrained section 1000a cannot fully expand due to the constraint, causing the constrained section 1000a of the stent to remain in a contracted state or in a semi-contracted state between the contracted state and the released state. The semi-contracted state specifically refers to the state of the constrained section 1000a, not the semi-constrained state of the entire stent.

The semi-constrained state of the stent is mainly achieved with the assistance of the first constraining member 2000. The first constraining member 2000 is disposed on the constrained section 1000a of the stent, providing a reference point for applying force at this constrained section 1000a. When the stent is accommodated between the outer catheter 5000 and the inner catheter of the delivery device, the first constraining member 2000 can utilize other constraining structures, such as a constraining wire, for connection. The constraining wire enters and exits through the stent lumen and the stent window 1100 of the stent, respectively, and after connecting to the first constraining member 2000, the constraining wire can constrain the constrained section 1000a of the stent with the assistance of the first constraining member 2000. For example, if the constraining wire connects the first constraining member 2000 to a certain position on the inner catheter, then the constrained section 1000a of the stent can be constrained to the inner catheter. When the outer catheter 5000 is removed, the unconstrained non-constrained section 1000b of the stent can expand normally, transitioning to the released state, while the constrained section 1000a cannot fully expand, resulting in the stent assuming a semi-constrained state. The constraint mentioned here primarily manifests as compression of the stent, causing deformation of a part of the stent structure, equivalent to compressing the constrained section 1000a of the stent. After compression, the constrained section 1000a conforms to the inner catheter, allowing positional movement of the stent in this case.

Referring to FIGS. 1 and 2, in an embodiment, taking a stent where the stent body 1000 includes two non-constrained sections 1000b and one constrained section 1000a distributed along the axial direction as an example, the constrained section 1000a is located between the two non-constrained sections 1000b. The number of stent window(s) 1100 is two, and the two stent windows 1100 are located at two junctions between the two non-constrained sections 1000b and the constrained section 1000a, respectively. In this case, the number of first constraining member(s) 2000 can also be selected as two, with the two first constraining members 2000 distributed along the axial direction on the constrained section 1000a. Still utilizing the constraining wire, the constraining wire enters the stent lumen from the right stent port in FIG. 1, forms a direct or indirect connection with a position on the inner catheter, exits through the first stent window 1100 on the right side, passes externally along the constrained section 1000a of the stent, connects to the two first constraining members 2000 along the way, then re-enters the stent lumen through the second stent window 1100 on the left side, and forms another direct or indirect connection with another position on the inner catheter. In this way, the constrained section 1000a of the stent can be constrained to the inner catheter. Referring to FIG. 2, when the outer catheter 5000 is removed, the unconstrained non-constrained sections 1000b of the stent can expand normally, transitioning to the released state, while the constrained section 1000a cannot fully expand. FIG. 2 shows that the constrained upper part of the constrained section 1000a cannot expand, while the unconstrained lower part of the constrained section 1000a can expand, thus causing the stent to form the semi-constrained state as shown in FIG. 2.

In other embodiments, when the numbers of the constrained section(s) 1000a, the stent window(s) 1100 and the first constraining member(s) 2000 of the stent are three, four or other quantities, the constrained section(s) 1000a of the stent can still be constrained in a manner similar to that shown in FIGS. 1 and 2, which is not limited here.

The first constraining member 2000 can take various forms. For example, in an embodiment, the first constraining member 2000 is a first constraining coil connected to the constrained section 1000a of the stent body 1000. The constraining wire can pass through the first constraining coil to achieve connection to the constrained section 1000a. Continuing to refer to FIG. 3, in an embodiment, each first constraining coil includes two unit coils 2000a. Both unit coils 2000a are connected to the constrained section 1000a of the stent body 1000. One unit coil 2000a in each first constraining coil is configured to thread through the other unit coil 2000a, and a constraining semi-annular channel is formed in a coil portion that threads through the other unit coil 2000a.

Continuing to refer to FIGS. 4 and 5, an inner catheter is further provided in the present disclosure, which includes an inner tube body 3000 and a second constraining member 4000. The inner tube body 3000 has an inner tube lumen extending therethrough in an axial direction, and a stent mounting section configured to mount the stent. The second constraining member 4000 is disposed on the exterior of the inner tube body 3000, and configured to assist in constraining the stent. Since the specific structure, functional principles, and technical effects of the stent have been detailed above, they will not be repeated here. Any technical content related to the stent can be referred to in the preceding descriptions. The second constraining member 4000 provided on the inner catheter is the cooperating structure that cooperates with the first constraining member 2000 to constrain the constrained section 1000a of the stent onto the inner catheter.

Referring to the embodiments shown in FIGS. 1, 2, and 4, the constraining wire enters the stent lumen of the stent from the right stent port in FIG. 1, connects to the first one of the second constraining members 4000 on the right side of the inner catheter, thereby connecting to the corresponding position on the inner catheter by means of this second constraining member 4000. Then, the constraining wire exits through the first stent window 1100 on the right side, passes externally along the constrained section 1000a of the stent, connects to the two first constraining members 2000 along the way, then re-enters the stent lumen of the stent through the second stent window 1100 on the left side, and connects to the second one of the second constraining members 4000 on the left side of the inner catheter, thereby connecting to the corresponding position on the inner catheter through this second constraining member 4000. This effectively constrains the constrained section 1000a of the stent to the inner catheter.

The second constraining member 4000 can take various forms. For example, in an embodiment, the second constraining member 4000 can be a component of any shape, provided with a constraining threading hole for the constraining wire to pass through and form a connection. Referring to FIG. 5, in an embodiment, the second constraining member 4000 can also directly be a second constraining coil. The second constraining coil is connected to the stent mounting section of the inner tube body 3000, and configured for the constraining wire to pass through and form a connection. The second constraining coil can be fixed to the inner catheter using a tube such as a heat-shrink tube. The constraining wire passes through the second constraining coil, allowing the inner catheter to drive the stent to move via the second constraining coil. The number of the second constraining members 4000 can be two or more, and the two or more constraining members are distributed along the axial direction on the stent mounting section, which is not limited here.

The present disclosure also provides a delivery assembly, which includes an outer catheter 5000, an inner catheter, a stent, and a third constraining member 6000. The outer catheter 5000 has an outer tube lumen extending therethrough in an axial direction. The inner catheter is movably assembled within the outer tube lumen of the outer catheter 5000. The stent is assembled between the outer catheter 5000 and the inner catheter, and is located at the stent mounting section of the inner catheter. The third constraining member 6000 is connected to the first constraining member 2000 and the second constraining member 4000, thereby constraining the constrained section 1000a of the stent body 1000 onto the inner catheter, such that the constrained section 1000a of the stent body 1000 is partially expanded. Since the specific structure, functional principles, and technical effects of the stent and the inner catheter have been detailed above, they will not be repeated here. Any technical content related to the stent and the inner catheter can be referred to in the preceding descriptions. The third constraining member 6000 is the cooperating structure that cooperates with the first constraining member 2000 and the second constraining member 4000 to constrain the constrained section 1000a of the stent to the inner catheter, i.e., the constraining wire mentioned above.

In an embodiment, the third constraining member 6000 is a constraining wire or other structure capable of smoothly passing through the stent window 1100 and the stent lumen. The third constraining member 6000 can possess a certain degree of flexibility, allowing it to deform within a certain range, as long as it can withstand the pulling force, and the shear force exerted on the third constraining member 6000 by the first constraining member 2000 and the second constraining member 4000 when under load without breaking. For example, the third constraining member 6000 can be made of a metal wire or the like, which is not limited here. The constraining wire, by virtue of its slender and flexible structural characteristics, can pass through the stent window 1100 of the stent and connect to the first constraining member 2000 and the second constraining member 4000, thereby constraining the constrained section 1000a of the stent body 1000 to the inner catheter, such that the constrained section 1000a of the stent body 1000 is partially expanded.

The inner catheter is movably assembled within the outer tube lumen of the outer catheter 5000, primarily meaning that the inner catheter can move axially and rotate circumferentially relative to the outer catheter 5000 within its outer tube lumen. The stent body with the stent window 1100 can be assembled between the inner catheter and the outer catheter 5000, i.e., the stent body is sleeved over the exterior of the inner catheter and housed inside the outer catheter 5000. The radial inward force applied by the outer catheter 5000 on the stent body keeps the stent body in the contracted state, stably assembled between the inner catheter and the outer catheter 5000 in the contracted state. Therefore, when the outer catheter 5000 is removed, the stent body, having lost the radial inward force applied by the outer catheter 5000, can expand and transition from the contracted state to the released state.

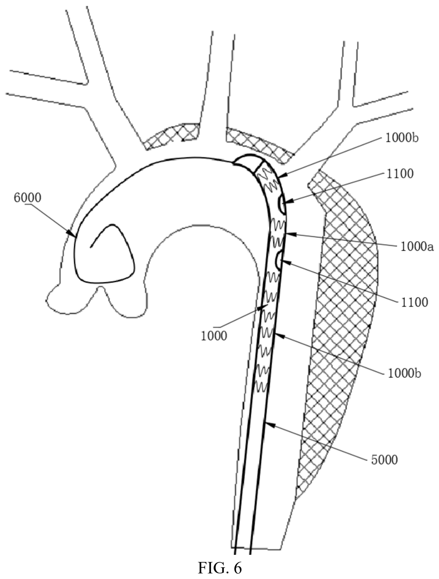

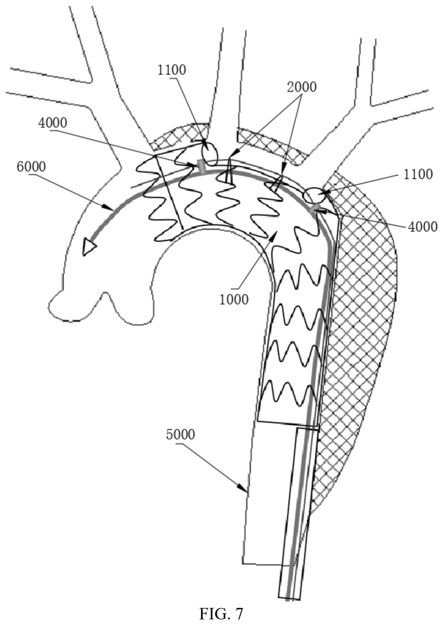

Referring to FIG. 4, the stent utilizes the cooperation of the first constraining member 2000, the second constraining member 4000, and the third constraining member 6000 to resist the expansion of the constrained section 1000a of the stent, i.e., it can configure the stent into a semi-constrained released state. This semi-constrained released state means that when the stent expands naturally upon removal of the radial inward force from the outer catheter 5000, the non-constrained sections 1000b of the stent can expand normally, while the constrained section 1000a cannot fully expand due to the combined action of the first constraining member 2000, the second constraining member 4000, and the third constraining member 6000, causing a part of the stent structure to remain constrained, either in the contracted state or in a state between the contracted and released states. As shown in FIG. 6, after the stent is assembled between the inner catheter and the outer catheter 5000 of the delivery device, it can enter a target position in a contracted state, for example, following the delivery device into the aorta. After reaching the aortic arch, as shown in FIG. 7, if the outer catheter 5000 is removed to release the stent, the non-constrained sections 1000b of the stent expand at this time, fixing within the blood vessel, while the third constraining member 6000 still constrains the constrained section 1000a of the stent. The non-release of the constrained section 1000a preserves blood flow in the aorta, while not completely blocking the branch vessel ostia. The reserved vascular space facilitates the downward super-selection of the stent window(s) 1100 on the stent from multiple branch vessels.

Meanwhile, because the third constraining member 6000, together with the first constraining member 2000 and the second constraining member 4000, clamps the constrained section 1000a of the stent onto the inner catheter, the constrained section 1000a of the stent does not fully conform to the blood vessel. In this case, moving the inner catheter can drive the stent to move together, allowing fine adjustment of the position of the stent window(s) 1100 to align it with the branch vessel position, facilitating the super-selection of the branch vessel. After the super-selection is completed, the third constraining member 6000 is removed, allowing the stent body 1000 to be fully released. Thus, the semi-constraining technique mentioned above can simplify the operation for stent fenestration, which can both ensure blood supply in the main lumen and facilitate the super-selection of the stent window(s) 1100 and branch vessels. Moreover, the non-constrained sections 1000b of the stent have already conformed to the blood vessel, so the stent will not easily shift during subsequent super-selection. The clamping technique of the stent also provides more operability for super-selection.

The present disclosure also provides a delivery device, which includes the delivery assembly as described in the above embodiments. Since the specific structure, functional principles, and technical effects of the delivery assembly have been detailed above, they will not be repeated here. Any technical content related to the delivery assembly can be referred to in the preceding descriptions.

The technical features in the above embodiments may be combined arbitrarily. For concise description, not all possible combinations of the technical features in the above embodiments are described. However, provided that they do not conflict with each other, all combinations of the technical features are to be considered to be within the scope of protection of the present disclosure.

The above-mentioned embodiments only describe several implementations of the present disclosure, and their description is specific and detailed, but should not be understood as a limitation on the protection scope of the present disclosure. It should be noted that, for a person of ordinary skill in the art, various variations and improvements can be further made without departing from the conception of the present disclosure, and these all fall within the protection scope of the present disclosure. Therefore, the protection scope of the present disclosure should be subject to the appended claims.

## Claims

1. A stent, comprising:
a stent body having a stent lumen extending therethrough in an axial direction, a stent window communicating with the stent lumen being defined on a surface of the stent body, the stent body having an expanded state and a constrained state, the stent body in the expanded state comprising a constrained section and a non-constrained section distributed along the axial direction, and the stent window being located at a junction between the constrained section and the non-constrained section when the stent body is in the constrained state; and
a first constraining member disposed on the constrained section of the stent body and configured to assist in constraining the constrained section of the stent body, such that the constrained section of the stent body is partially expanded.

2. The stent according to claim **1,** wherein the stent body comprises two non-constrained sections and one constrained section distributed along the axial direction, the constrained section is located between the two non-constrained sections, the stent window comprises two stent windows, and the two stent windows are located at two junctions between the two non-constrained sections and the constrained section, respectively.

3. The stent according to claim 1, wherein the first constraining member comprises two first constraining members, and the two first constraining members are spaced apart along the axial direction on the constrained section.

4. The stent according to claim 1, wherein the first constraining member is a first constraining coil connected to the constrained section of the stent body.

5. The stent according to claim 4, wherein the first constraining coil comprises two unit coils, both of which are connected to the constrained section of the stent body, and one of the unit coils in the first constraining coil is configured to thread through the other unit coil, forming a constraining semi-annular channel in a portion of the unit coil threaded through the other unit coil.

6. An inner catheter, comprising:
an inner tube body having an inner tube lumen extending therethrough in an axial direction, and having a stent mounting section configured to mount the stent of any one of claims 1 to 5; and
a second constraining member disposed on the inner tube body and configured to assist in constraining the stent of any one of claims 1 to 5.

7. The inner catheter according to claim 6, wherein the second constraining member is provided with a constraining threading hole configured for a constraining wire to pass through.

8. The inner catheter according to claim 6, wherein the second constraining member comprises two second constraining members, and the two constraining members are spaced apart along the axial direction on the stent mounting section.

9. The inner catheter according to claim 6, wherein the second constraining member is a second constraining coil connected to the stent mounting section of the inner tube body.

10. A delivery assembly, comprising:
an outer catheter having an outer tube lumen extending therethrough in an axial direction;
the inner catheter of any one of claims 6 to 9, wherein the inner catheter is movably assembled within the outer tube lumen of the outer catheter;
the stent of any one of claims 1 to 5, wherein the stent is assembled between the outer catheter and the inner catheter, and the stent is located at the stent mounting section of the inner catheter; and
a constraining assembly configured to connect to the first constraining member, thus constraining the constrained section of the stent body onto the inner catheter, such that the constrained section of the stent body is partially expanded.

11. The delivery assembly according to claim 10, wherein the constraining assembly comprises the second constraining member on the inner catheter and a third constraining member.

12. The delivery assembly according to claim 11, wherein the third constraining member is a constraining wire, the constraining wire is threaded through the stent window of the stent and connected to the first constraining member and the second constraining member, thus constraining the constrained section of the stent body onto the inner catheter, such that the constrained section of the stent body is partially expanded.

13. A delivery device, comprising the delivery assembly according to any one of claims 10 to 12.
